# EUROPEAN PATENT APPLICATION

(11) **EP 1 398 312 A1**
(43) Date of publication of application: **17.03.2004**
(21) Application number: 02020643.9
(22) Date of filing: 13.09.2002
(51) Int. Cl.: C07D 307/32

(54) **Beta-substituted-gamma-butyrolactones and a process for preparation thereof**

(71) Applicant: Uh, Hong-Sun, Kangnam-gu, 135-942 Seoul (KR)
(72) Inventor: Uh, Hong-Sun, Kangnam-gu, 135-942 Seoul (KR)
(74) Representative: Lönnqvist, Gunnel Solveig Kristina

(57) **Abstract**

The present invention is related to a process for the preparation of (S)- or (R)-β-substituted-γ-butyrolactones or a racemic mixture thereof. β-substituted-γ-succinic anhydrides are converted by zinc borohydride in a compatible solvent selected from tetrahydrofuran, methylene chloride, and ethyl acetate to the corresponding β-substituted-γ-butyrolactones. The β-substituted-γ-butyrolactones obtainable in chrystallin form are useful in the production of pharmaceuticals, agrochemicals, flavors and fragrances. Also disclosed are novel β-substituted-γ-butyrolactones.

## Description

The present invention is related to a process for the preparation of (S)- or (R)-β-substituted-γ-butyrolactones or a racemic mixture thereof. β-substituted-γ-succinic anhydrides are converted by zinc borohydride in a compatible solvent selected from tetrahydrofuran, methylene chloride, dichloromethane and ethyl acetate to the corresponding β-substituted-γ-butyrolactones. The β-substituted-γ-butyrolactones are obtainable in chrystalline form and are useful in the production of pharmaceuticals, agrochemicals, flavors and fragrances. Also disclosed are novel β-substituted-γ-butyrolactones.

Methods for preparing β-substituted-γ-butyrolactones are described in the following patents US 3,024,250 (Klein et al.), US 3,868,370 (Smith), US 3,997,569 (Powell), US 4,105,674 (De Thomas et al.), US 4,155,919 (Ramioulle et al.), US 4,772,729 (Rao), US 4,940,805 (Fisher et al.), US 5,292,939 (Hollingsworth), US 5,319,110 (Hollingsworth), US 5,374,773 (Hollingsworth), US 5,502,217 (Fuchikami et al.) and in the following articles Bailey et al., J. Org.Chem. 35 (10) 3574(1970), McAlees et al., J.C.S. Perkin I.2037 (1977), McGarvey et al., J. Am. Chem. Soc., 108, 4943-4952 (1986), Corey et al., J. Am. Chem. Soc., 100, 1942-1943 (1978).

The first synthesis of (S)-(-)-β-benzyloxy-γ-butyrolactone starting from ℓ-malic acid was published by Barton et al. in Tetrahedron Lett. 1982, 651. The use of ℓ-malic acid as a precursor for the preparation of (S)-β-hydroxy-γ-butyrolactone was published by Hollingsworth in the patent US 5,808,107. However, the lithium borohydride made *in situ* from sodium borohydride, and lithium chloride is still expensive and it is difficult to measure the exact quantity necessary for the reduction. Furthermore, an excess of lithium borohydride may overreduce the ester to a triol.

Accordingly, the use of alkali metal borohydride, particularly sodium borohydride, as a reducing agent for the conversion of the anhydride to lactone is in general known in the prior art, but the γ-butyrolactones obtained by the known process are viscous syrupy products. Much labor is required to purify them to commercially satisfactory quality.

Therefore, there is a need for a further improved process for the preparation of (S)- or (R)-β-hydroxy-γ-butyrolactone from l-malic acid. In particular, the object of this invention is to find improved methods for preparing of (S)- or (R)-β-hydroxy and other substituted-γ-butyrolactones from l-malic acid.

The characteristic features of the present invention are as set out in the claims.

As said above the present invention is based on a method first disclosed by Barton, et al., (Tetrahedron Lett. 1982,651) and Hollingsworth (US 5,808,107).

A route of the syntesis is shown in Scheme I. 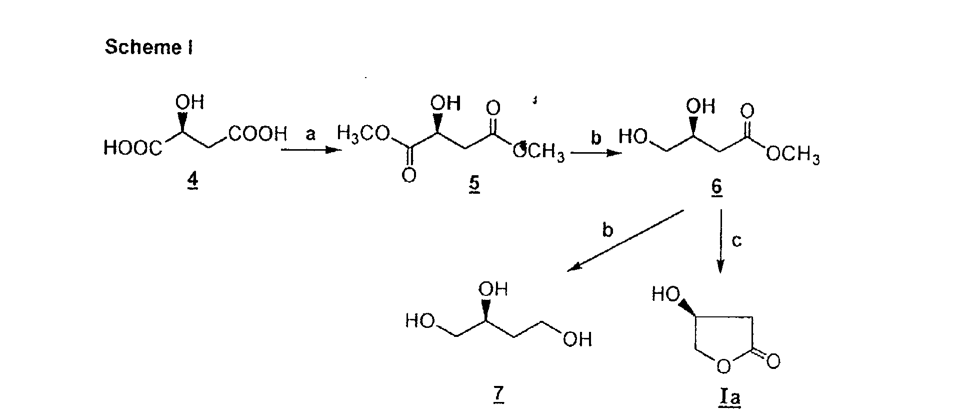 Conditions : (a) CH₃OH, H⁺ ; (b) LiBH₄ ; (c) H⁺

In said sheme 4 is ℓ-malic acid
5 is an ester of the ℓ-malic acid
6 is a deesterified diol of 5
7 is 6 reduced to a triol
Ia is γ-butyrolactone

As said above the use of alkali metal borohydride, particularly sodium borohydride, as a reducing agent for the conversion of the anhydride to lactone is known in the prior art, but is replaced by zink borohydride in the present invention.

Alternatively, (S)-β-acetoxy-succinic anhydride 2, made from ℓ-malic acid 4, can be reduced by sodium borohydride to (S)-β-acetoxy-butyrolactone Ic in good yield as shown in Scheme II.

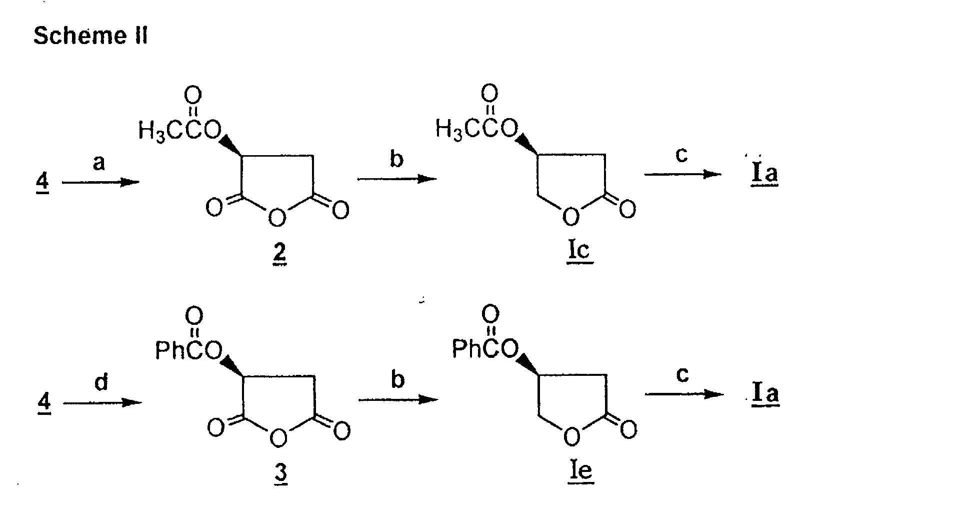

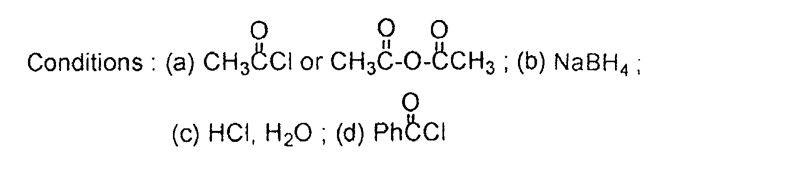

In Scheme II 4 is ℓ-malic acid
2 is (S)-β-acetoxy-succinic anhydride
3 is (S)-β-benzoyloxy succinic anhydride
Ia is γ-butyrolactone
Ic is (S)-β-acetoxy-γ-butyrolactone
Ie is (S)-β-benzoyloxy-γ-butyrolactone

The γ-butyrolactone Ia and Ic are both viscous syrupy products and their purification to commercially satisfactory quality is laborious. The present inventors have shown that if the β-hydroxy group of ℓ-malic acid is blocked by a benzoyl group, the resulting (S)-β-benzoyloxy succinic anhydride 3 is a crystalline compound and can be reduced by sodium borohydride to give crystalline (S)-β-benzoyloxy-γ-butyrolactone Ie. This product can be recrystallized to meet the quality requirements for production of pharmaceuticals.

The present inventors found that zinc borohydride is a better reducing agent for the reduction of (S)-β-acetoxy succinic anhydride 2 and (S)-β-benzoyloxy-succinic anhyride 3 to the corresponding γ-butyrolactones Ic and Ie, giving better qualities and yields using the syntesis routes shown in Scheme II. Zinc borohydride is also less expensive than other alkali borohydrides.

β-Substituted-γ-butyrolactones of (R)-series are also prepared by the same chemistry applied to (S)-series. Zinc borohydride reduction of the anhydrides is preferred to sodium borohydride with respect to yields and purities.

Crude (S)- or (R)-β-hydroxy-γ-butyrolactones are water-soluble and viscous compounds. These compounds are as said above very difficult to purify using conventional methods, including for example simple or high-vacuum distillations. The present inventors demonstrated that when (S)- or (R)-β-hydroxy-γ-butyrolactones were benzoylated, the resulting (S)- or (R)-β-hydroxy-γ-butyrolactones Ie and If became crystalline compounds which were easily purified by recrystallizations in a compatible solvent (Scheme III).

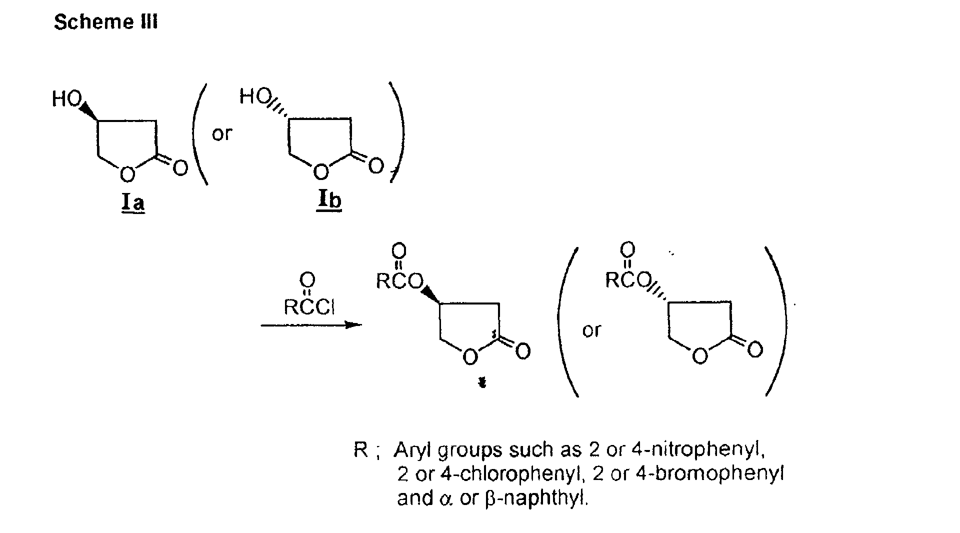

Accordingly, the present invention relates to an improved process for the preparation of β-substituted-γ-butyrolactones or racemic mixtures thereof having the general formula (I), 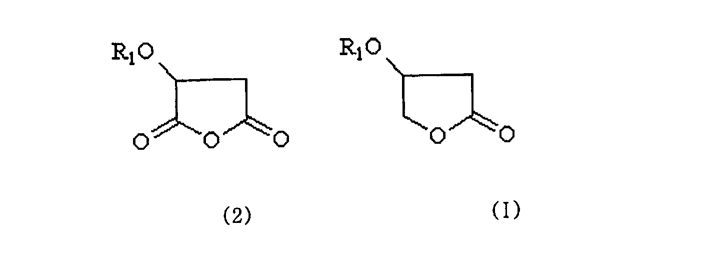 wherein R₁ is hydrogen, acetyl, benzyl, benzoyl, 2- or 4-nitrophenylcarbonyl, 2- or 4-bromophenyl carbonyl, 2- or 4-chlorophenylcarbonyl, or 1- or 2-naphtylcarbonyl. The (S)- or (R)-β-substituted-λ-butyrolactones of the formula (I) above of the present invention are obtainable by converting β-substituted-γ-succinic anhydrides of the formula (2) in the presence of zinc borohydride in a compatible solvent selected from tetrahydrofuran, methylene chloride, dichloromethane and ethyl acetate.

β-substituted-γ-butyrolactones of the formula (I) of the present invention have chiral center at β-position. Therefore, the compounds of the present invention exist as (S)-isomer(I-1), (R)-isomer(I-2) and racemic mixture thereof.

The compounds of the present invention are shown below. 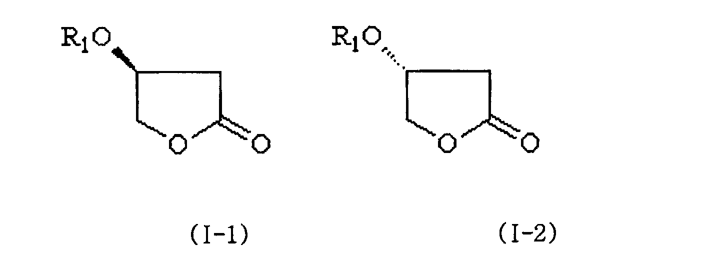 wherein, R₁ is benzoyl, 2-or 4-nitrophenylcarbonyl, 2- or 4-bromophenyl carbonyl, 2- or 4-chlorophenylcarbonyl, or 1- or 2-naphthylcarbonyl.

The compounds of the present invention can be illustrated as (S)-β-hydroxy-γ-butyrolactone (Ia), (R)-β-hydroxy-γ-butyrolactone (Ib), (S)-β-acetoxy-γ-butyrolactone (Ic), (R)-β-acetoxy-γ-butyrolactone (Id), (S)-β-benzyloxy-γ-butyrolactone, (R)-β-benzyloxy-γ-butyrolactone, (S)-β-benzoyloxy-γ-butyrolactone (Ie), (R)-β-benzoyloxy-γ-butyrolactone (If), (S)-β-[2-nitro(or 4-nitro)phenyl]carbonyloxy-γ-butyrolactone, (R)-β-[2-nitro(or 4-nitro) phenyl]carbonyloxy-γ-butyrolactone, (S)-β-[2-chloro-(or 4-chloro)phenyl]carbonyloxy-γ-butyrolactone, (R)-β- [2-chloro-(or 4-chloro)phenyl]carbonyloxy-γ-butyrolactone, (S)-β-[2-bromo-( or 4-bromo) phenyl]carbonyloxy-γ-butyrolactone, (R)-β-[2-bromo-( or 4-bromo)phenyl]carbonyloxy-γ-butyrolactone, (S)-β-[1-naphthyl-(or 2-naphthyl)]-γ-butyrolactone, (R)-β-[1-naphthyl (or 2-naphthyl)]carbonyloxy -γ-butyrolactone, or racemic mixtures thereof.

Among the above compounds, compounds (Ia), (Ib), (Ic) and (Id) are known compounds and compounds (Ie) and (If) etc., are novel compounds.

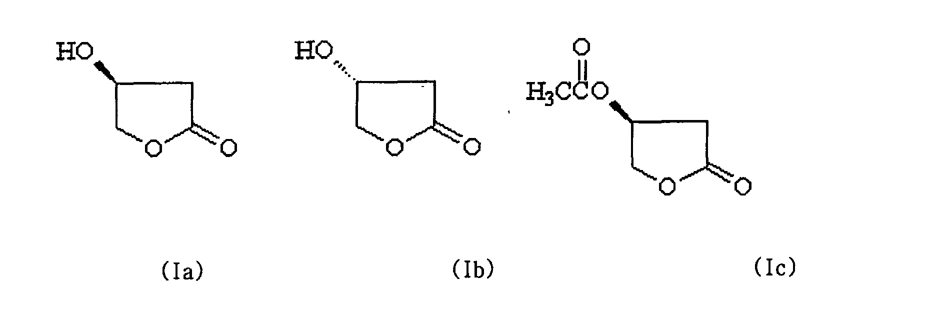

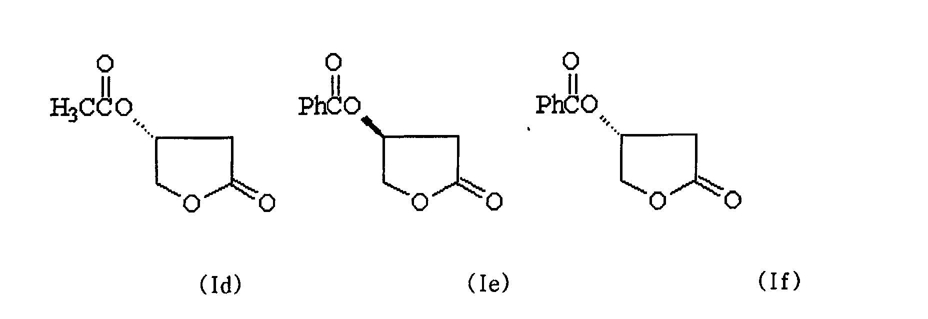

The above mentioned β-substituted-γ-butyrolactones are the important intermediates for the production of many pharmaceuticals, agrochemicals, flavors and fragrances.

Novel compounds of (Ie), (If) and derivatives thereof including (S)-[2-nitro(or 4-nitro)phenyl]carbonyloxy-γ-butyrolactone, (R)-[2-nitro(or 4-nitro)phenyl]carbonyloxy-γ-butyrolactone, (S)-β-[2-chloro-( or 4-chloro) phenyl]carbonyloxy-γ-butyrolactone, (R)-β-[2-chloro-(or 4-chloro)phenyl]carbonyloxy-γ-butyrolactone, (S)-β-[2-bromo-( or 4-bromo)phenyl]carbonyloxy-γ-butyrolactone, (R)-β-[2-bromo-(or 4-bromo)phenyl]carbonyloxy-γ-butyrolactone, (S)-β-[1-naphthyl-(or 2-naphthyl)]carbonyloxy-γ-butyrolactone, (R)-β-[1-naphthyl (or 2-naphthyl)]carbonyloxy-γ-butyrolactone, or racemic mixtures thereof newly prepared by the present invention have very good physical and chemical properties other than the known compounds (Ia), (Ib), (Ic) and (Id). Therefore, the novel compounds of the present invention can be used as important intermediates for the production of many pharmaceuticals, agrochemicals, flavors and fragrances.

### Example 1

### Preparation of (S)-β-Acetoxysuccinic Anhydride 2.

Acetyl chloride (8.8 g, 111.9 mmole) was added to ℓ-malic acid (5g-37.3 mmole) with stirring under nitrogen atmosphere and refluxed at 55-60°C., 3 hrs. When the evolution of hydrogen chloride gas stopped, the reaction mixture was cooled to room temperature, treated with THF (10ml) and then stirred for 10 min. The solvent and excess acetyl chloride were removed under a rotary evaporator. Diethyl ether (20ml) was added to the reaction mixture and stirred to obtain a white solid, 5.5g (92.2% yield); m.p. 56-57°C IR(KBr): 1871 and 1792 cm⁻¹ (C=0, anhydride) and 1746 cm⁻¹ (C=0, acetoxy group).

### Example 2

### Preparation of (S)-β-Benzoyloxysuccinic-Anhydride 3.

Benzoyl chloride (15.7 g, 111.9 mmole) was added to 1-malic (5.0g, 37.3 mmole) in 10 ml anhydrous THF with stirring at room temperature and heated to 80-85°C, for 3 hours. When the evolution of hydrogen chloride gas stopped, THF was distilled off under a rotary evaporator and diethyl ether (20ml) was added and stirred. A white solid was filtered, washed with 5ml of diethyl ether and dried to obtain 6.5 g (79.1% yield); m.p. 159-160°C.

IR (KBr) 1862 and 1789 cm⁻¹ (C=0, anhydride) and 1708 cm⁻¹(C=0, benzoyl).

### Example 3

### Preparation of (S)-β-Acetoxy-γ-Butyrolactone Ic.

To Zn(BH₄)₂ (1.44g,15.8mmole) in 24ml THF, (S)-β-Acetoxy-γ-succinic-anhydride 2 (5.0g, 31.6mmol) in 20ml THF was added below 5°C over 1 hour and stirred at room temperature for 3 hours. The reaction mixture was cooled again below 5°C, treated with 5ml of 6N HCI and stirred for half an hour. THF was removed under a rotary evaporator and oily water layer was extracted twice with 50ml of ethyl acetate. The combined ethyl acetate was dried over sodium sulfate, filtered and evaporated to yield 4.8g (85%) of yellow oil.

IR (KBr): 1738 (C=0, acetoxy) and 1784 cm⁻¹ (C=0, lactone).

### Example 4

### Preparation of (S)-β-Benzoyloxy-γ-Butyrolactone Ie

To Zn(BH₄)₂ (0.62g,6.8mmole) in 10ml THF, (S)-β-benzoyloxy-γ-succinic-anhydride (3.0g, 13.6mmol) dissolved in 20ml THF was added slowly below 5°C over one hour and stirred at room temperature for 3 hours. The reaction mixture was cooled again below 5°C, treated with 5ml of 6N HCI solution and stirred for half an hour. 20ml of THF was removed under a rotary evaporator and kept at 5°C over night in a refrigerator.

0.2g of white solid was obtained which was unknown impurity. The filtrate was concentrated to obtain 2.8g of white solid. The solid was dissolved in 100ml methylene chloride and extracted with 20ml of 1N HCl solution three times. The evaporation of methylene chloride yielded a white solid, 2.6g (88.0% yield); m.p. 73-74°C.

IR (KBr): 1716 (C=0, benzoyloxy) and 1770 cm⁻¹ (C=0, lactone).

### Example 5

### Preparation of (R )-β-Benzoyloxy-γ-Butyrolactone If

By using (R)-β-benzoyloxy-γ-succinic anhydride instead of (S)-β-benzoyloxy-γ-succinic anhydride of Example 4, the titled compound was obtained.

Other compounds can be prepared by analogy method of example 3 or 4 by using proper starting materials.

It is intended that the foregoing description would be only illustrated for the present invention and that the present invention would be limited only by the hereinafter-appended.

## Claims

1. A process for the preparation of butyrolactone or racemic mixture thereof of the formula (I) **characterized in that** the butyrolactone is a (S)- or (R)-β-substituted-λ-butyrolactone of the formula (I), obtainable by converting β-substituted-γ-succinic anhydrides of the formula (2) in the presence of zinc borohydride in a compatible solvent selected from tetrahydrofuran, methylene chloride, dichloromethane and ethyl acetate, 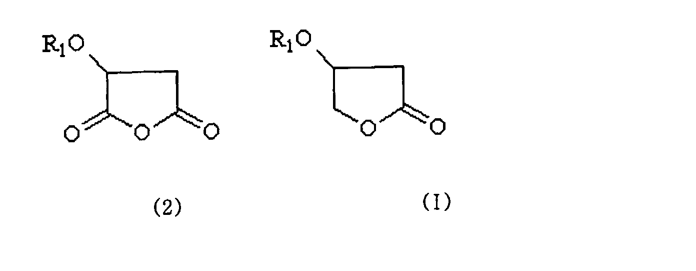 wherein R₁ is hydrogen, acetyl, benzyl, benzoyl, 2-or 4-nitrophenylcarbonyl, 2- or 4-bromophenyl carbonyl, 2-or 4-chlorophenylcarbonyl, or 1- or 2-naphtylcarbonyl.

2. A butyrolactone or racemic mixture thereof **characterized in that** the butyrolactone is a (S)- or (R)-β-substituted-λ-butyrolactone of the formulas (I-1) or (I-2), 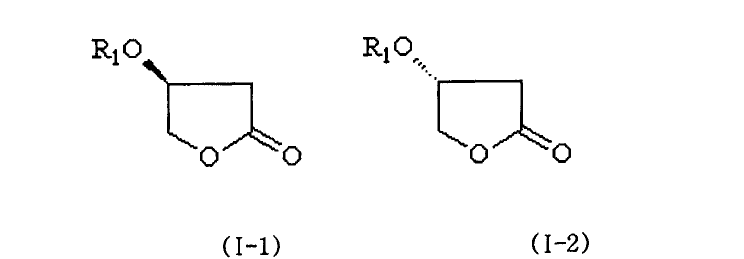 wherein, R₁ is benzoyl, 2-or 4-nitrophenylcarbonyl, 2- or 4-bromophenyl carbonyl, 2- or 4-chlorophenylcarbonyl, or 1- or 2-naphthylcarbonyl.

3. The butyrolactone according to claim 2 **characterized in that** the butyrolactone is a (S)- or (R)-β-substituted-λ-butyrolactone selected from (S)-β-benzoyloxy-γ-butyrolactone (Ie), (R)-β-benzoyloxy-γ-butyrolactone (If), (S)-β-[2-nitro(or 4-nitro)phenyl]carbonyloxy-γ-butyrolactone, (R)-β-[2-nitro(or 4-nitro)phenyl]carbonyloxy-γ-butyrolactone, (S)-β[2-chloro-( or 4-chloro)phenyl]carbonyloxy-γ-butyrolactone, (R)-β-[2-chloro-( or 4-chloro)phenyl]carbonyloxy-γ-butyrolactone, (S)-β-[2-bromo-( or 4-bromo)phenyl]carbonyloxy-γ-butyrolactone, (R)-β-[2-bromo-(or 4-bromo)phenyl]-γ-butyrolactone, (S)-β-[1-naphthyl-(or 2-naphthyl)]-γ-butyrolactone, (R)-β-[1-naphthyl (or 2-naphthyl)]-γ-butyrolactone.

4. A butyrolactone according to claim 2 **characterized in that** the butyrolactone is a (S)-β-benzoyloxy-γ-butyrolactone (Ie).

5. A butyrolactone according to claim 2 **characterized in that** the butyrolactone is a (R)-β-benzoyloxy-γ-butyrolactone (If).

6. A butyrolactone according to claim 2 **characterized in that** the butyrolactone is a (S)-β-benzoyloxycarbonyl-γ-butyrolactone.

7. A butyrolactone according to claim 2 **characterized in that** the butyrolactone is a (R)-β-benzoyloxycarbonyl-γ-butyrolactone.
